# EUROPEAN PATENT APPLICATION

(11) **EP 4 406 471 A1**
(43) Date of publication of application: **31.07.2024**
(21) Application number: 22871819.3
(22) Date of filing: 08.09.2022
(51) Int. Cl.: A61B 1/307

(54) **VISUAL URETEROSCOPE**

(30) Priority: 22.09.2021 CN 202111102333
(71) Applicant: Ningbo Xinwell Medical Technology Co., Ltd., Cixi, Zhejiang 315334 (CN); The First Affiliated Hospital of Ningbo University, Ningbo, Zhejiang 315000 (CN)
(72) Inventor: SHAN, Jian, Cixi, Zhejiang 315334 (CN); CHENG, Yue, Ningbo, Zhejiang 315000 (CN); HUANG, Junjun, Cixi, Zhejiang 315334 (CN); CHEN, Qingye, Cixi, Zhejiang 315334 (CN); WU, Hailiang, Cixi, Zhejiang 315334 (CN); FANG, Li, Ningbo, Zhejiang 315000 (CN); LI, Qiang, Ningbo, Zhejiang 315000 (CN)
(74) Representative: Haseltine Lake Kempner LLP
(86) International application number: PCT/CN2022/117842
(87) International publication number: WO 2023/045771

(57) **Abstract**

A visual ureteroscope (1), comprising a ureteroscope body (10) and a ureteroscope tip (20), which is arranged in the ureteroscope body (10). The ureteroscope tip (20) comprises a tip portion (21) and an image capture device (22), wherein the tip portion (21) is arranged at a front end of a ureteroscope tube (11) of the ureteroscope body (10); the tip portion (21) has a camera end face (2101) and an aspiration end face (2102), which is located in the front of the camera end face (2101); the tip portion (21) comprises an aspiration hole (211), which is in communication with an aspiration channel (101) of the ureteroscope body (10), and an operating hole (212) for an operating component (12) to pass therethrough, the aspiration hole (211) axially extending forwards from the aspiration channel (101) to the aspiration end face (2102) to form an aspiration opening (2110), and the operating hole (212) obliquely extending forwards such that the operating component (12) that passes out from the operating hole (212), obliquely extends forwards to extend out of the tip portion (21); and the image capture device (22) comprises a camera (221), which is mounted on the camera end face (2101) of the tip portion (21), and the aspiration opening (2110) and a head portion (120) of the operating component (12) that passes out from the operating hole (212) are both within the field of view of the camera (221).

## Description

### TECHNICAL FIELD

The present disclosure relates to the technical field of medical devices, and in particular to a visual ureteroscope.

### BACKGROUND

Urinary calculi are a common disease. According to statistics, the prevalence of urolithiasis in adults is up to 6.5%, and its 5-year recurrence rate is up to 50%, which is increasing year by year and seriously threatens people's health. In recent years, with the development of minimally invasive treatment technology, ureteroscopy has become an important treatment for such diseases. The existing ureteroscope equipment for urinary system lithotomy mainly includes rigid ureteroscope and flexible ureteroscope. The rigid ureteroscope is only suitable for diagnosis and treatment of ureteral calculi and other diseases because its main body is relatively hard and can not be bent, while the flexible ureteroscope has gradually become an important treatment for urinary calculi. Taking the ureteroscope as an example, the conventional flexible ureteroscope still has deficiencies in image acquisition, calculus crushing and residual calculus cleaning. For example, the calculus can be seen at a turning point of the renal pelvis, but an optical fiber cannot aim, or an image is blocked by an inner wall of the renal pelvis after aiming, and a state of crushed calculus can not be observed, or a head portion of the flexible ureteroscope has only a small channel, which can only crush calculus through the fiber optic, but cannot efficiently remove residual calculus out of the body, resulting in problems such as low surgical efficiency.

In order to solve the above problems, some clinical experts have proposed to use the principle of negative pressure suction to suck the crushed calculus out of the body in time. For example, as shown in FIG. 1, Chinese utility model patent CN212574841U has provides a self-perfusion and discharging ureteroscope 1P, which has an advantage that a camera 10P, an perfusion port 20P, a fiber channel opening 30P and a suction channel opening 40P are provided on a front end surface of the ureteroscope, and the suction channel opening 40P is located behind the camera 10P. The camera 10P collects an end operating image to observe a light to break the calculus, and then the water flow is used to wash the calculus and suck it to form an operation cycle, so as to improve the calculus removal efficiency.

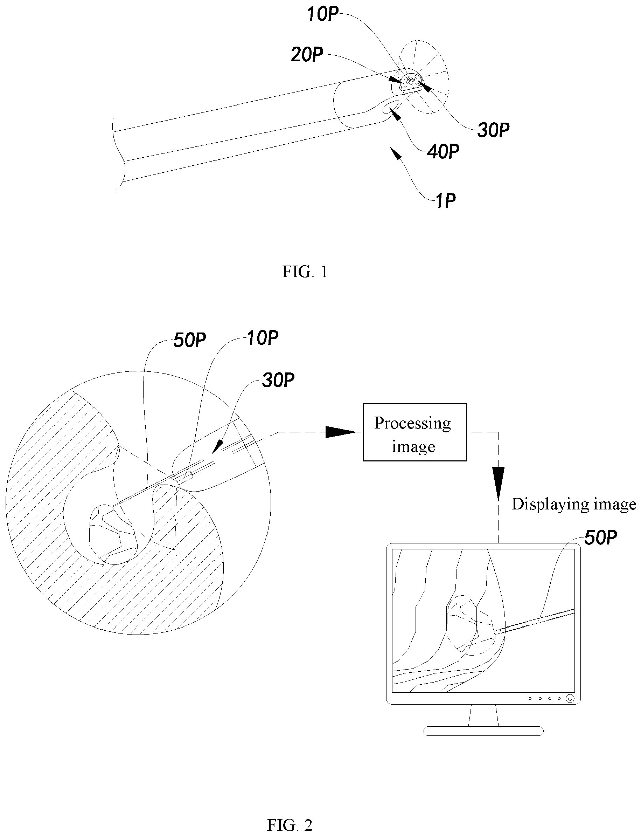

However, during a practical test, due to factors such as a position of the camera, a direction of the optical fiber, a shape of the suction channel opening and an arrangement of the perfusion channel, there is a blind area when the calculus is crushed, which makes it difficult for a doctor to give corresponding correct operation feedback. For example, as shown in FIG. 2, since the camera 10P is located in front of the suction channel opening 40P, the suction channel opening 40P is out of a field of view of the camera 10P. Therefore, whether the crushed calculus enters the suction channel opening 40P, or whether the suction channel opening 40P is blocked, cannot be observed, which brings great safety risk to the operation.

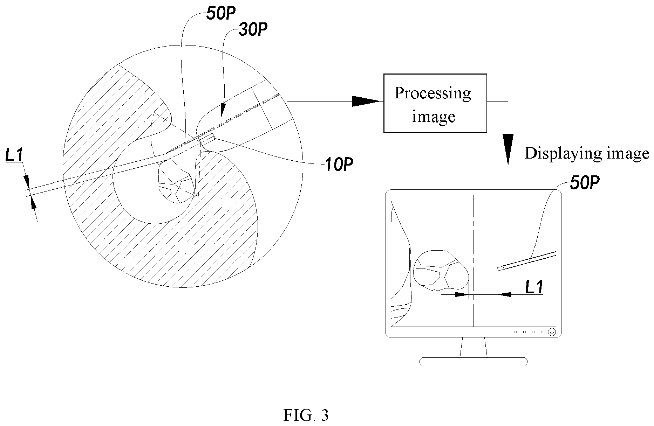

In addition, as shown in FIG. 2 and FIG. 3, although an image of the optical fiber 50P extending from the fiber channel opening 30P can be observed, since the optical fiber 50P extending from the fiber channel opening 30P extends toward the front of the ureteroscope along a direction parallel to an optical axis of the camera 10P, the head portion of the optical fiber 50P can only touch the front of the ureteroscope. In this way, once the calculus is located at the turning point in the renal pelvis, when the camera 10P captures the calculus, the head portion of the optical fiber 50P is blocked by the renal pelvis and can not aim, while when the head portion of the optical fiber 50P aims at the calculus, the field of view of the camera 10P is blocked by the renal pelvis, and the state of the calculus cannot be observed, that is, the self-perfusion and discharging ureteroscope 1P has the problem of being visible but not being broken, and being broken but being invisible, which will bring great risk to the operation.

### SUMMARY

### Technical problems

### Solution to the problem

### Technical solutions

An advantage of the present disclosure is to provide a visual ureteroscope, which can observe a suction opening of a suction hole while observing a head portion of an optical fiber, which is helpful to make an accurate judgment on a surgical status in time.

Another advantage of the present disclosure is to provide a visual ureteroscope. In an embodiment of the present disclosure, the visual ureteroscope enables both a suction opening and a head portion of the optical fiber to be within a field of view of the image capturing device, so as to observe a phenomenon such as whether a crushed calculus enters the suction opening of a suction hole or whether the suction opening of the suction hole is blocked while observing the optical fiber breaking the calculus.

Another advantage of the present disclosure is to provide a visual ureteroscope. In an embodiment of the present disclosure, the visual ureteroscope can be released from a limitation of a field of view of an image capturing device by sucking prior to capturing image, so as to ensure that both a suction opening and a head portion of an optical fiber are located within the field of view of the image capturing device without increasing a field of view of the image capturing device.

Another advantage of the present disclosure is to provide a visual ureteroscope. In an embodiment of the present disclosure, the visual ureteroscope is capable of guiding an optical fiber obliquely forward through an inclined operation hole, so that a position of a head portion of the optical fiber can be changed only by rotating the ureteroscope, so as to observe the head portion of the optical fiber when breaking calculus.

Another advantage of the present disclosure is to provide a visual ureteroscope. In an embodiment of the present disclosure, a central axis of the operation hole in the visual ureteroscope is located on a meridian image plane of the camera, so that an image of the optical fiber extending through the operation hole coincides with a visual center axis of the camera, and the image of the optical fiber is located at a lower part of the image captured by the camera, which is convenient to conform to an observation habit of human beings.

Another advantage of the present disclosure is to provide a visual ureteroscope. In an embodiment of the present disclosure, the visual ureteroscope is capable of extending an optical fiber out of a suction opening to extend forward, so as to simultaneously observe a state of a head portion of the optical fiber and the suction opening to ensure smooth and efficient operation of the visual ureteroscope.

Another advantage of the present disclosure is to provide a visual ureteroscope. In an embodiment of the present disclosure, a suction end surface and an optical fiber of the visual ureteroscope are on a same side of the camera, and both the suction end surface and the optical fiber extend obliquely forward, so as to achieve a visible breaking effect while observing the suction opening.

Another advantage of the present disclosure is to provide a visual ureteroscope. In an embodiment of the present disclosure, the meridian image plane of the camera of the visual ureteroscope passes through the suction opening of the suction hole, so that a visual center axis of the camera passes through the suction opening, which facilitates observation of the state of the suction opening.

Another advantage of the present disclosure is to provide a visual ureteroscope. In an embodiment of the present disclosure, the visual ureteroscope is able to visualize an operation state of a tip portion through an inclined suction opening, which helps a doctor to master the surgical state.

Another advantage of the present disclosure is to provide a visual ureteroscope. In an embodiment of the present disclosure, the visual ureteroscope enables the suction opening to cover the entire front end surface of the tip portion as much as possible, so as to increase a size of the suction opening and reduce the risk of the suction opening being blocked.

Another advantage of the present disclosure is to provide a visual ureteroscope. In an embodiment of the present disclosure, the visual ureteroscope can make the suction opening within the field of view of the camera through a front and inclined suction opening, so as to facilitate observation of the state of the suction opening.

Another advantage of the present disclosure is to provide a visual ureteroscope. In order to achieve the above advantages, a complex structure or design is not required in the present disclosure. Therefore, the present disclosure successfully and effectively provides a solution that not only provides a simple visual ureteroscope, but also increases the practicability and reliability of the visual ureteroscope.

In order to achieve at least one of the above and other advantages and objects, the present disclosure provides a visual ureteroscope, which includes:
a ureteroscope body including a ureteroscope tube and an operating component mounted to the ureteroscope tube, and the ureteroscope body being provided with a suction channel extending axially along the ureteroscope tube; and
a ureteroscope tip provided on the ureteroscope body, the ureteroscope tip including:
   a tip portion provided at a front end of the ureteroscope tube, the tip portion having an imaging end surface and a suction end surface located in front of the imaging end surface, wherein the tip portion is provided with a suction hole in communication with the suction channel and an operating hole through which the operating component extends, the suction hole extends axially forward from the suction channel to the suction end surface to form a suction opening, and the operating hole extends obliquely forward, so that the operating component extending out of the operating hole extends obliquely forward out of the tip portion; and
   an image capturing device including a camera mounted on the imaging end surface of the tip portion, wherein both the suction opening of the suction hole and a head portion of the operating component extending out of the operating hole are located within a field of view of the camera.

According to an embodiment of the present application, the suction end surface of the tip portion extends obliquely forward from the imaging end surface of the tip portion.

According to an embodiment of the present application, the suction end surface of the tip portion extends obliquely downward and forwardly from the imaging end surface.

According to an embodiment of the present application, the suction end surface of the tip portion includes a concave end surface extending inwardly and arcuately from the imaging end surface and a convex end surface extending outwardly and arcuately from the concave end surface, and the concave end surface of the suction end surface is tangent to the imaging end surface.

According to an embodiment of the present application, an operating opening of the operating hole of the tip portion faces the suction opening of the suction hole of the tip portion.

According to an embodiment of the present application, a central axis of the operating hole of the tip portion intersects a central axis of the suction hole.

According to an embodiment of the present application, the operating hole of the tip portion extends obliquely from top to bottom.

According to an embodiment of the present application, the operating hole extends obliquely forward and inward from a rear end surface of the tip portion to an inner wall surface of the suction hole, so as to form the operating opening at the inner wall surface of the suction hole.

According to an embodiment of the present application, a central axis of the operating hole of the tip portion is located in a meridian image plane of the camera.

According to an embodiment of the present application, a central axis of the suction hole of the tip portion is located in the meridian image plane of the camera.

According to an embodiment of the present application, an optical axis of the camera intersects the central axis of the operating hole.

According to an embodiment of the present application, the ureteroscope body is further provided with an operating channel extending axially within the ureteroscope tube, the operating component extends through the operating channel, and the operating hole of the tip portion is in communication with the operating channel of the ureteroscope body.

According to an embodiment of the present application, the suction channel and the operating channel of the ureteroscope body are in communication with each other.

According to an embodiment of the present application, the ureteroscope body further includes a perfusion channel extending axially in the ureteroscope tube, the tip portion further is provided with a perfusion hole in communication with the perfusion channel, and the perfusion hole extends from a rear end surface of the tip portion to an outer peripheral side surface of the tip portion, so as to form one or more perfusion openings at the outer peripheral side surface of the tip portion.

According to an embodiment of the present application, the perfusion channel of the ureteroscope body has an annular structure to wrap the suction channel and the operating channel.

According to an embodiment of the present application, the perfusion channel of the ureteroscope body has an irregular shaped structure, and the perfusion channel wraps the suction channel.

According to an embodiment of the present application, the perfusion channel and the operating channel of the ureteroscope body are in communication with each other, so as to form a complete annular channel surrounding the suction channel.

According to an embodiment of the present application, the image capturing device further includes at least one light source, and the light source and the camera are adjacently mounted to the tip portion.

According to an embodiment of the present application, the operating component is an optical fiber configured to emit a laser to perform a calculus crushing operation.

Further objects and advantages of the present application will become more fully apparent from an understanding of the following description and drawings.

These and other objects, features, and advantages of the present application will become more fully apparent from the following detailed description, drawings, and claims.

### Beneficial effects of the present disclosure

### Beneficial effects

### BRIEF DESCRIPTION OF THE DRAWINGS

### Description of the drawings

FIG. 1 is a partial schematic view of a self-perfusion and discharging ureteroscope in the prior art.
FIG. 2 and FIG. 3 are schematic views of the application of the self-perfusion and discharging ureteroscope, respectively.
FIG. 4 is a state view of a visual ureteroscope according to an embodiment of the present disclosure.
FIG. 5 is a partial enlarged schematic view of the visual ureteroscope according to the above embodiment of the present disclosure.
FIG. 6 is a schematic view of the application of the visual ureteroscope according to the above embodiment of the present disclosure.
FIG. 7 is a partial cross-sectional view of the visual ureteroscope according to the above embodiment of the present disclosure.
FIG. 8 is a top view of the visual ureteroscope according to the above embodiment of the present disclosure.
FIG. 9 is a partial cross-sectional view of a ureteroscope body of the visual ureteroscope according to the above embodiment of the present disclosure.
FIG. 10 is a schematic view of a calculus crushing operation using the visual ureteroscope according to the above embodiment of the present disclosure.
FIG. 11 is a first embodiment of the ureteroscope body of the visual ureteroscope according to the above embodiment of the present disclosure.
FIG. 12 is a second embodiment of the ureteroscope body of the visual ureteroscope according to the above embodiment of the present disclosure.
FIG. 13 is a third embodiment of the ureteroscope body of the visual ureteroscope according to the above embodiment of the present disclosure.
FIG. 14 is a fourth embodiment of the ureteroscope body of the visual ureteroscope according to the above embodiment of the present disclosure.

### DETAILED DESCRIPTION OF THE EMBODIMENTS

### Embodiments of the preset disclosure

The following description is used to disclose the invention to enable a person skilled in the art to implement the invention. The preferred embodiments in the following description are by way of example only, and other obvious variations will occur to those skilled in the art. The general principles of the present disclosure, as defined in the following description, may be applied to other embodiments, variations, modifications, equivalents and alternatives without departing from the spirit and scope of the present disclosure.

It should be understood by those skilled in the art that the terms "longitudinal", "lateral", "upper", "lower", "front," back", "left", "right", "vertical", "horizontal", "top", "bottom", "inner," outer" and the like in the disclosure of the present disclosure indicate orientations or positional relationships based on the orientations or positional relationships shown in the drawings, which is intended for convenience and simplicity of description only and is not intended to indicate or imply that devices or elements referred to must have or be constructed or operate in a particular orientation. Therefore, the above terms should not be construed as limitations of the present disclosure.

In the present disclosure, the term "a" or "an" in the claim and description should be understood to mean "one or more", that is, in an embodiment, the number of element may be one, and in other embodiments, the number of element may be multiple. The term "a" or "an" is not to be construed as only one, and the term "a" or "an" is not to be construed as a limitation on the number of element, unless it is explicitly stated in the present disclosure that the number of element is only one.

In the description of the present disclosure, it should be understood that the terms "first", "second", etc., are used for descriptive purposes only and are not to be construed as indicating or implying relative importance. In the description of the present disclosure, it should be noted that, unless otherwise specified and limited, "connected" and "connection" should be interpreted in a broad sense, for example, they may be fixed connection, detachable connection or integral connection. They can be a mechanical connection or an electrical connection. They can be connected directly or indirectly through medium. Those skilled in the art can understand the specific meaning of the above terms in the present disclosure according to the specific situation.

In the description of this specification, reference terms "one embodiment", "some embodiments", "example", "specific example", or "some examples" or the like means that a specific feature, structure, material, or characteristic described in connection with the embodiment or example is included in at least one embodiment or example of the present disclosure. In this specification, the schematic expression of the above terms need not be directed to the same embodiments or examples. Moreover, the specific feature, structure, material, or characteristics described may be combined in any suitable manner in any one or more embodiments or examples. Furthermore, different embodiments or examples and features of different embodiments or examples described in this specification may be combined and incorporated by those skilled in the art without being mutually contradictory.

### Overview of the application

As described in the background, during a practical test of the existing self-perfusion and discharging ureteroscope, due to factors such as a position of the camera, a direction of the optical fiber, a shape of the suction channel opening and an arrangement of the perfusion channel, there is a blind area when a calculus is crushed, which makes it difficult for a doctor to give corresponding correct operation feedback. For example, since the camera is located in front of the suction channel opening, the suction channel opening is out of a field of view of the camera. Therefore, whether a crushed calculus enters the suction channel opening, or whether the suction channel opening is blocked, cannot be observed. In addition, since the optical fiber extending from the fiber channel opening extends toward the front of the ureteroscope along a direction parallel to an optical axis of the camera, the head portion of the optical fiber can only touch the front of the ureteroscope. In this way, once the calculus is located at the turning point in the renal pelvis, when the camera captures the calculus, the head portion of the optical fiber is blocked by the renal pelvis and can not aim, while when the head portion of the optical fiber aims at the calculus, the field of view of the camera is blocked by the renal pelvis, and the state of the calculus cannot be observed, that is, the self-perfusion and discharging ureteroscope has the problem of being visible but not being broken, and being broken but being invisible, which will bring great risk to the operation.

Specifically, a technical concept of the present application is to creatively design a tip portion of the ureteroscope in full consideration of the characteristics of calculus crushing operation and a practical application scene of the ureteroscope. In this way, when a minimally invasive treatment surgery is performed, not only an operation state of a suction opening can be observed in real time, but also a visible and breaking effect can be achieved, which helps a doctor to make an accurate judgment on the operation status in time and improve the efficiency of crushing calculus.

In view of this, the present application provides a visual ureteroscope, which includes a ureteroscope body and a ureteroscope tip provided on the ureteroscope body. The ureteroscope body includes a ureteroscope tube and an operating portion mounted on the ureteroscope tube, and the ureteroscope body is provided with a suction channel extending axially along the ureteroscope tube. The ureteroscope tip includes: a tip portion, wherein the tip portion is provided at a front end of the ureteroscope tube, and the tip portion has an imaging end surface and a suction end surface located in front of the imaging end surface, the tip portion is provided with a suction hole in communication with the suction channel and an operating hole through which the operating portion extends, the suction hole extends axially forward from the suction channel to the suction end surface to form a suction opening, and the operating hole extends obliquely forward, so that the operating portion extending out of the operating hole extends obliquely forward out of the tip portion; and an image capturing device, wherein the image capturing device includes a camera mounted on the imaging end surface of the tip portion, and both the suction opening of the suction hole and a head portion of the operating portion extending out of the operating hole are located within a field of view of the camera.

### Exemplary embodiments

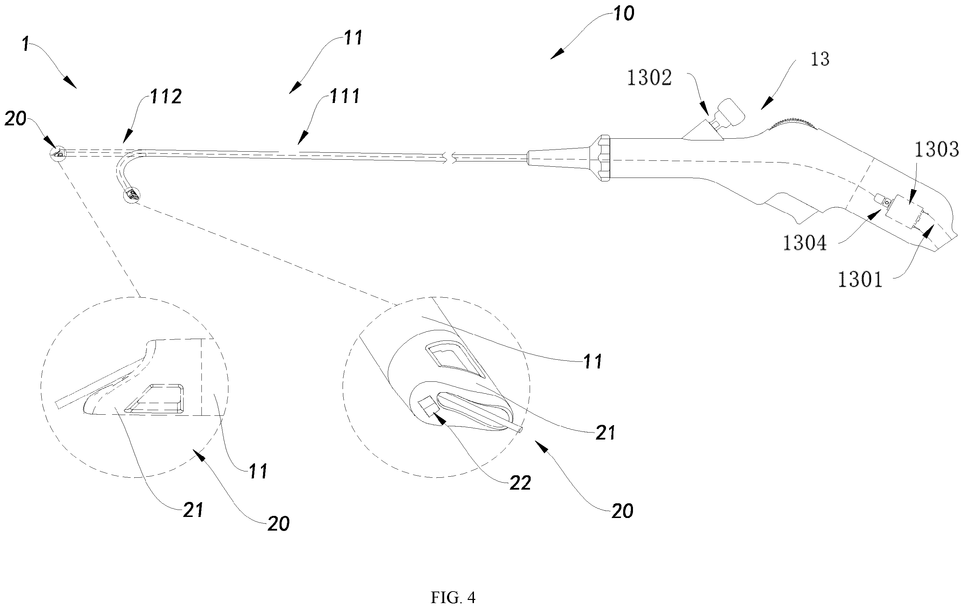

Referring to FIGS. 4 to 10 of the accompanying drawings of the present disclosure, an embodiment of the present disclosure provides a visual ureteroscope 1, which can be applied to the treatment of urinary calculi and other diseases. Those skilled in the art should understand that, for the convenience of description, a direction of the visual ureteroscope 1 entering the body is defined as front, and a direction outside the body is rear.

Specifically, as shown in FIG. 4 to FIG. 6, the visual ureteroscope 1 may include a ureteroscope body 10 and a ureteroscope tip 20. The ureteroscope body 10 includes a ureteroscope tube 11 and an operating component 12 mounted to the ureteroscope tube 11, and the ureteroscope body 10 is provided with a suction channel 101 extending axially along the ureteroscope tube 11 configured to discharge fluid and crushed calculus. The ureteroscope tip 20 is provided on the ureteroscope body 10, and the ureteroscope tip 20 may include a tip portion 21 and an image capturing device 22. The tip portion 21 is provided at a front end of the ureteroscope tube 11, and has an imaging end surface 2101 and a suction end surface 2102 located in front of the imaging end surface 2101. The tip portion 21 is provided with a suction hole 211 in communication with the suction channel 101 and an operating hole 212 through which the operating component 12 extends. The suction hole 211 extends axially forward from the suction channel 101 to the suction end surface 2102 to form a suction opening 2110, and the operating hole 212 extends obliquely, so that the operating component 12 extending through the operating hole 212 extends obliquely forward out of the tip portion 21. The image capturing device 22 includes a camera 221 mounted on the imaging end surface 2101 of the tip portion 21, and both the suction opening 2110 of the suction hole 211 and a head portion 120 of the operating component 12 extending through the operating hole 212 are both within a field of view of the camera 221.

It should be understood that the imaging end surface 2101 and the suction end surface 2102 of the tip portion 21 of the present application cooperatively form a front end surface of the tip portion 21, and a rear end surface of the tip portion 21 is connected to the ureteroscope tube 11. In this way, the suction hole 211 extends from the rear end surface of the tip portion 21 to the front end surface of the tip portion 21, so that the suction hole 211 extends forward to the suction end surface 2102 to form the suction opening 2110 while being in communication with the suction channel 101, and liquid or crushed calculus in a human organ can firstly enter the suction hole 211 from the suction opening 2110 and then be discharged out of the body through the suction channel 101.

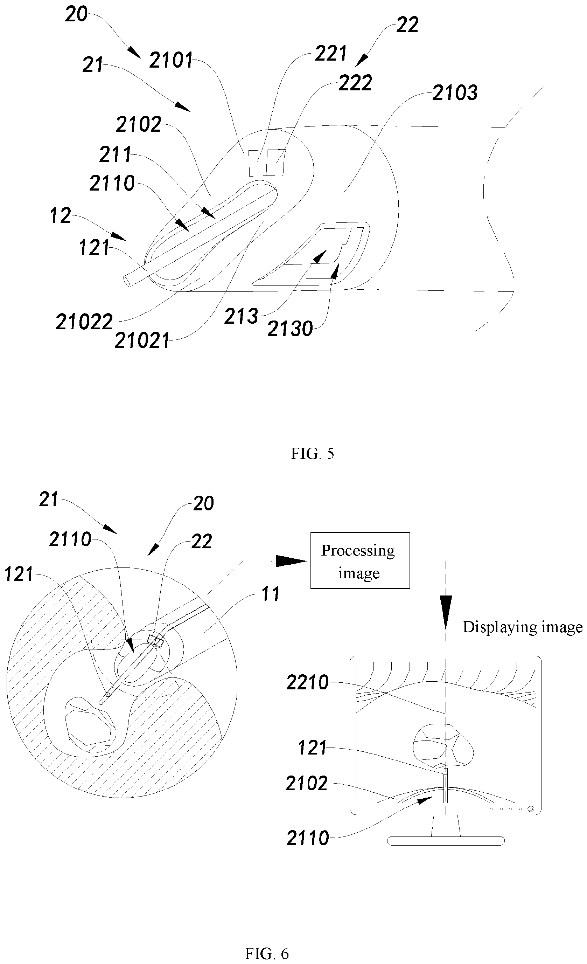

It should be noted that, the camera 221 and the suction opening 2110 of the suction hole 211 of the ureteroscope tip 20 are respectively located at the imaging end surface 2101 and the suction end surface 2102 of the tip portion 21, and the suction end surface 2102 is located in front of the imaging end surface 2101. Therefore, as shown in FIG. 5 and FIG. 6, the suction opening 2110 of the suction hole 211 will be located in front of the image capturing device 22, so as to achieve the technical effect of sucking prior to capturing image, so as to ensure that the suction opening 2110 of the suction hole 211 is partially or completely located within the field of view of the camera 221, and the operating state of the suction opening 2110 can be observed in real time, such as whether the crushed calculus enters the suction opening 2110, or whether the suction opening 2110 is blocked, etc. At the same time, since the operating hole 212 extends obliquely forward, the operating component 12 extending through the operating hole 212 extends obliquely forward into the field of view of the camera 221, so that the camera 221 can simultaneously capture the suction opening 2110 and the head portion of the operating component 12, which helps a doctor to make an accurate judgment on the operation status in time.

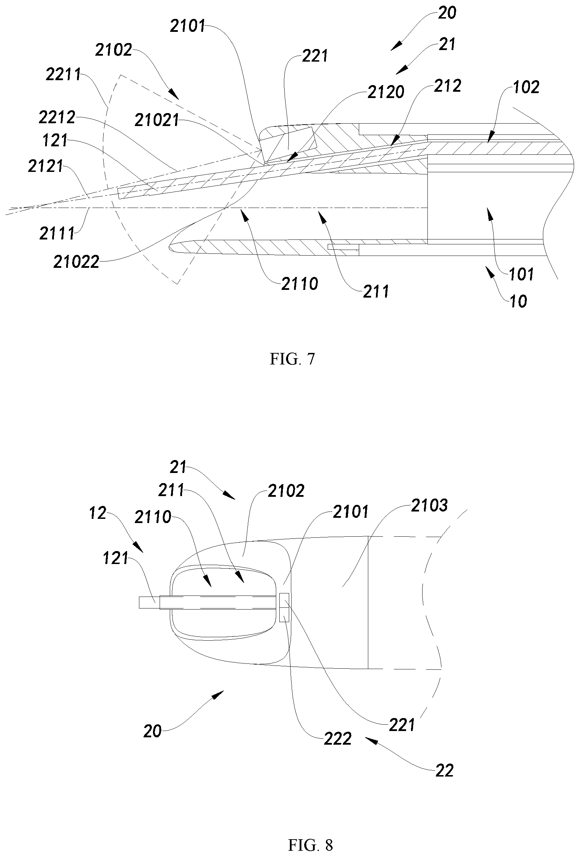

Specifically, as shown in FIGS. 5 and 7, the operating component 12 may be implemented as, but is not limited to, an optical fiber 121, so that laser is emitted through the optical fiber 121 to perform a calculus crushing operation. Those skilled in the art should understood that a type of the operating component 12 can be different according to different application scenarios of the ureteroscope 1, and an operator can select the type of the operating component 12 according to needs.

In addition, when the ureteroscope 1 is used for diagnosis and treatment, the ureteroscope tip 20 will be inserted into the human body, that is, the ureteroscope tip 20 will be in a lightless environment. Therefore, as shown in FIG. 5 and FIG. 8, the image capturing device 22 generally further include at least one light source 222 for capturing images. The light source 222 is configured to emit light to irradiate an object to be captured, such as a renal pelvis lumen or the suction opening 2110, etc., and the camera 221 is configured to receive the light reflected back by the object to be captured, so as to capture an image of the object to be captured, so that a captured image data is transmitted outside the body to be displayed in a monitor, which is convenient for the doctor and other personnel to observe the situation in the body.

For example, as shown in FIG. 5 and FIG. 8, both the camera 221 and the light source 222 are mounted on the imaging end surface 2101 of the tip portion 21, and the light source 222 is located adjacent to the camera 221, that is, the camera 221 and the light source 222 are adjacently mounted on the tip portion 21, which helps to ensure that the light emitted by the light source 222 can be better received by the camera 221 to obtain image information after being reflected by the object to be captured.

Specifically, the light source 222 may be implemented as, but not limited to, an LED or a cold light source, and a number of the light source 222 may be one or more than one. In addition, the light source 222 may be located on one side or both sides of the camera 221, and may be configured according to needs and space, which is not described herein again. It should be understood that the camera 221 may be implemented as, but not limited to, an imaging module composed of a lens assembly and a CMOS image sensor, and may also be implemented as other types of imaging modules, as long as image information can be collected.

According to the above embodiment of the present application, as shown in FIG. 5 and FIG. 7, the suction end surface 2102 of the tip portion 21 extends obliquely forward from the imaging end surface 2101 of the tip portion 21, so that the image capturing device 22 mounted on the imaging end surface 2101 is at the rear, and the suction opening 2110 formed on the suction end surface 2102 is at the front, and the suction opening 2110 is an inclined opening, so as to ensure that the suction opening 2110 is partially or completely located within the field of view of the image capturing device 22. It should be understood that, in the above embodiment of the present application, the suction end surface 2102 of the tip portion 21 is implemented as an inclined surface. Certainly, in other embodiments of the present application, the suction end surface 2102 of the tip portion 21 may also be implemented as a flat surface, and in this case, the imaging end surface 2101 of the tip portion 21 may be located at a notch of the suction end surface 2102 or in the suction hole 211, so that the image capturing device 22 can still acquire the image of the suction opening 2110.

For example, as shown in FIG. 5 and FIG. 7, the suction end surface 2102 of the tip portion 21 extends obliquely downward and forwardly from the imaging end surface 2101, so that the imaging end surface 2101 is located above the suction end surface 2102, so as to achieve the arrangement effect of the camera being placed above and the suction hole being placed below. In this way, the image of the suction opening 2110 will be located at a lower part of a picture captured by the camera 221, which conforms to the observation habit of people, and helps the doctor to operate the visual ureteroscope 1 based on the image feedback. It should be understood that the upper, lower, left and right mentioned in the present application are defined according to the camera 221 placed in an upright position, that is, the upper, lower, left and right mentioned in the present application are respectively corresponding to the upper, lower, left, and right of the camera 221 when it is placed upright.

Preferably, as shown in FIG. 5 and FIG. 7, the suction end surface 2102 of the tip portion 21 includes a concave end surface 21021 extending inwardly and arcuately from the imaging end surface 2101, so as to prevent the concave end surface 21021 from blocking the field of view of the imaging end surface 2101, which is conducive to ensuring that the suction opening 2110 located at the suction end surface 2102 is within the field of view of the camera 221. At the same time, a size of the suction opening 2110 of the tip portion 21 on the concave end surface 21021 can be rapidly expanded to a maximum inner diameter of the suction channel 101, which helps to prevent the suction opening 2110 from being blocked by large crushed calculus.

More preferably, the suction end surface 2102 of the tip portion 21 further includes a convex end surface 21022 extending outwardly and arcuately from the concave end surface 21021, so that the tip portion 21 has a blunt head structure, while preventing the tip portion 21 from forming a sharp head portion, which helps prevent the tip portion 21 from damaging human organs.

Most preferably, the convex end surface 21022 of the suction end surface 2102 is tangent to the concave end surface 21021 of the suction end surface 2102, and the concave end surface 21021 of the suction end surface 2102 is tangent to the imaging end surface 2101, so that the concave end surface 21021 of the suction end surface 2102 smoothly extends from the imaging end surface 2101 to the convex end surface 21022, so as to ensure that the tip portion 21 has a smooth end surface, and further to prevent the tip portion 21 from damaging human organs.

It should be noted that an angle of the field of view of the camera 221 may be, but is not limited to, 120°. According to the arrangement of the present application, the visual ureteroscope 1 of the present application can visualize the ureteroscope tip 20 without increasing the angle of the field of view of the camera 221, which helps to observe whether the suction opening 2110 of the tip portion 21 is blocked, or whether the crushed calculus enters the suction opening 2110. Certainly, in other embodiments of the present application, the angle of the field of view of the camera 221 may also be implemented as other angles.

According to the above embodiment of the present application, as shown in FIG. 5 to FIG. 7, the operating opening 2120 of the operating hole 212 of the tip portion 21 may face the suction opening 2110 of the suction hole 211 of the tip portion 21, so that the optical fiber 121 extending through the operating hole 212 can extend from the suction opening 2110 of the tip portion 21, which helps to ensure that an extended portion of the optical fiber 121 can be within the field of view of the camera 221, so as to facilitate the observation of a position and state of the extended portion of the optical fiber 121.

It should be noted that since the operating hole 212 of the tip portion 21 extends obliquely forward, and the operating opening 2120 of the operating hole 212 corresponds to the suction opening 2110 of the suction hole 211 of the tip portion 21, the optical fiber 121 extending through the operating hole 212 can extend obliquely forward from the suction opening 2110 of the tip portion 21. In this way, when the optical fiber 121 is operated to extend from the suction opening 2110 of the suction hole 211, laser emitted from the optical fiber 121 can break calculus in a human organ to perform the calculus crushing operation. When the optical fiber 121 is operated to retract into the suction opening 2110 of the suction hole 211, the laser emitted from the optical fiber 121 is released in the suction hole 211. At this time, if the crushed calculus is blocked in the suction hole 211, the released holmium laser will break the blocked crushed calculus to achieve the effect of unclogging the suction hole 211.

Preferably, a central axis 2121 of the operating hole 212 of the tip portion 21 intersects a central axis 2111 of the suction hole 211, so that the suction opening 2110 and the operating component 12 extending from the suction hole 211 can be simultaneously captured by the camera 221.

More preferably, the operating hole 212 of the tip portion 21 extends obliquely from top to bottom, so that the optical fiber 121 extending through the operating hole 212 extends out of the suction opening 2110 obliquely downward from an upper side of the suction hole 211, so that an image of the optical fiber 121 is also located at a lower portion of a picture captured by the camera 221, which conforms to the observation habit of people.

For example, as shown in FIG. 7, the operating hole 212 of the tip portion 21 may extend obliquely forward and inward from the rear end surface of the tip portion 21 to an inner wall surface of the suction hole 211, so as to form the operating opening 2120 at the inner wall surface of the suction hole 211. That is, the operating hole 212 is implemented as an inclined hole with respect to the suction hole 211, so that the optical fiber 121 extending through the operating hole 212 can extend from an inner wall of the suction hole 211, so as to prevent the optical fiber 121 from blocking the crushed calculus or fluid from entering the suction channel 101 through the suction hole 211 and being discharged.

Preferably, as shown in FIG. 6 and FIG. 7, the central axis 2121 of the operating hole 212 of the tip portion 21 is located in a meridian image plane 2211 of the camera 221. That is, the optical fiber 121 protruding from the suction opening 2110 extends in the meridian image plane 2211 of the camera 221, so that the image of the optical fiber 121 extends substantially along a visual central axis 2210 of the camera 221, such that it will not be blocked by tissue at the turning point of the renal pelvis lumen, and a visible breaking effect is achieved.

More preferably, the central axis 2111 of the suction hole 211 of the tip portion 21 is also located in the meridian image plane 2211 of the camera 221, so that the image of the suction opening 2110 of the suction hole 211 is symmetrical about the visual central axis 2210 of the camera 221, so as to facilitate observing the operating state of the suction opening 2110 to the greatest extent.

It should be noted that an optical axis 2212 of the camera 221 of the present application may be parallel to the central axis 2111 of the suction hole 211, or may not be parallel to the central axis 2111 of the suction hole 211, as long as it is ensured that the suction opening 2110 of the suction hole 211 is completely or partially located within the field of view of the camera 221.

In addition, an angle between the central axis 2121 of the operating hole 212 and the optical axis 2212 of the camera 221 of the present application may be less than a half-field-of-view angle of the camera 221, so as to ensure that the operating component 12 extending through the operating hole 212 can be located within the field of view of the camera 221.

Preferably, the optical axis 2212 of the camera 221 is deviated towards the central axis 2111 of the suction hole 211, which helps to reduce the requirement of the camera with a large field of view for the visual ureteroscope 1. That is, a camera with a small field of view can be selected to achieve the effect of simultaneously observing the suction opening 2110 and the operating component 12.

More preferably, as shown in FIG. 7, the optical axis 2212 of the camera 221 intersects the central axis 2121 of the operating hole 212, so that the optical fiber 121 extending through the operating hole 212 can extend to a visual center of the camera 221. In this way, only the visual ureteroscope 1 needs to be controlled so that the calculus is located in a visual center area of the camera 221, the optical fiber 121 can aim at the calculus to break the calculus, which helps to improve the calculus crushing efficiency and reduce the risk of accidental injury to the tissue in the renal pelvis.

According to the above embodiment of the present application, as shown in FIG. 7, the ureteroscope body 10 may further include an operating channel 102 extending axially in the ureteroscope tube 11 to allow the operating component 12 to extend through. The operating hole 212 of the tip portion 21 is in communication with the operating channel 102, so that the operating portion 212 inserted into the operating channel 102 can extend through the operating hole 212 to extend out of the tip portion 21 obliquely forward to be located within the field of view of the camera 221. It should be understood that the operating component 12 inserting into the operating channel 102 and the operating hole 212 is movable, such that a length of the operating component 12 extending out of the operating hole 212 can be increased or decreased by pushing and pulling the operating component 12, so as to facilitate breaking calculus at different positions in the renal pelvis. Exemplarily, the optical fiber 121 may be movably mounted in the operating channel 102, such that the optical fiber 121 can extend out of or retract into the operating hole 212 by pushing and pulling the optical fiber 121.

In other words, the optical fiber 121 may be movably mounted in the operating channel 102 to be able to extend out of or retract into from the suction opening 2110 of the suction hole 211 by pulling the optical fiber 121. In this way, when the optical fiber 121 is operated to extend from the suction opening 2110 of the suction hole 211, the laser emitted from the optical fiber 121 can break calculus in a human organ to perform the calculus crushing operation, while when the optical fiber 121 is operated to retract into the suction opening 2110 of the suction hole 211, the laser emitted from the optical fiber 121 is released in the suction hole 211. At this time, if the crushed calculus is blocked in the suction hole 211, the released holmium laser will break the blocked crushed calculus to achieve the effect of unclogging the suction hole 211.

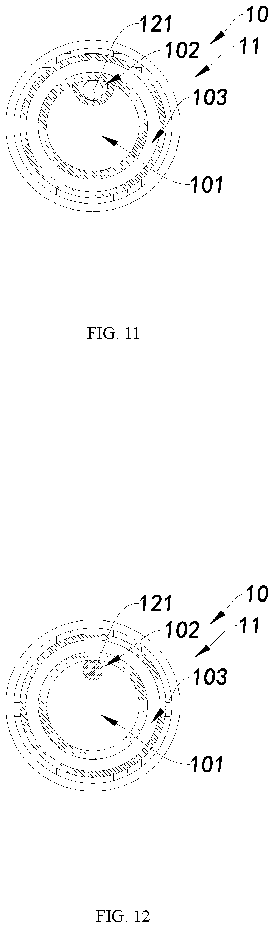

Exemplarily, in a first embodiment of the present application, as shown in FIG. 7 and FIG. 11, the suction channel 101 and the operating channel 102 of the ureteroscope body 10 may be independent from each other. That is, the suction channel 101 and the operating channel 102 extend between a front end and a rear end of the ureteroscope body 10, respectively. It should be understood that, since the suction channel 101 and the operating channel 102 are independent from each other, the operating component 12 (such as the optical fiber 121) mounted in the operating channel 102 will not enter the suction channel 101, so as to prevent the operating component 12 from interfering with the movement of the fluid or the crushed calculus in the suction channel 101 and avoid congestion of the suction channel 101.

It should be noted that, in a second embodiment of the present application, as shown in FIG. 12, the suction channel 101 and the operating channel 102 of the ureteroscope body may be in communication with each other, that is, the suction channel 101 and the operating channel 102 may be implemented as a same channel. However, since the operating hole 212 extends obliquely from a side wall of the suction hole 211 of the tip portion 21 from the outside to the inside, the optical fiber 121 extending through the operating hole 212 will extend against an inner wall of the suction channel 101. That is, the optical fiber 121 firstly extends against the inner wall of the suction channel 101, and then extends obliquely out of the suction opening 2110 of the suction hole 211 through the operating hole 212. At this time, the optical fiber 121 can still avoid interfering with the movement of the fluid or the crushed calculus in the suction channel 101 to a certain extent, so as to avoid blocking in the suction channel 101. It should be understood that, when the suction channel 101 and the operating channel 102 of the ureteroscope body are the same channel, the structure of the ureteroscope body 10 will be simplified to the greatest extent, which helps to reduce the manufacturing difficulty and the manufacturing cost of the ureteroscope body 10. Meanwhile, once the suction channel 101 of the ureteroscope body 10 is blocked by crushed calculus, the optical fiber 121 can be pulled, so that an end portion of the optical fiber 121 is located at a crushed calculus blockage in the suction channel 101, so that the laser emitted from the optical fiber 121 breaks the blocked crushed calculus to unclog the suction channel 101.

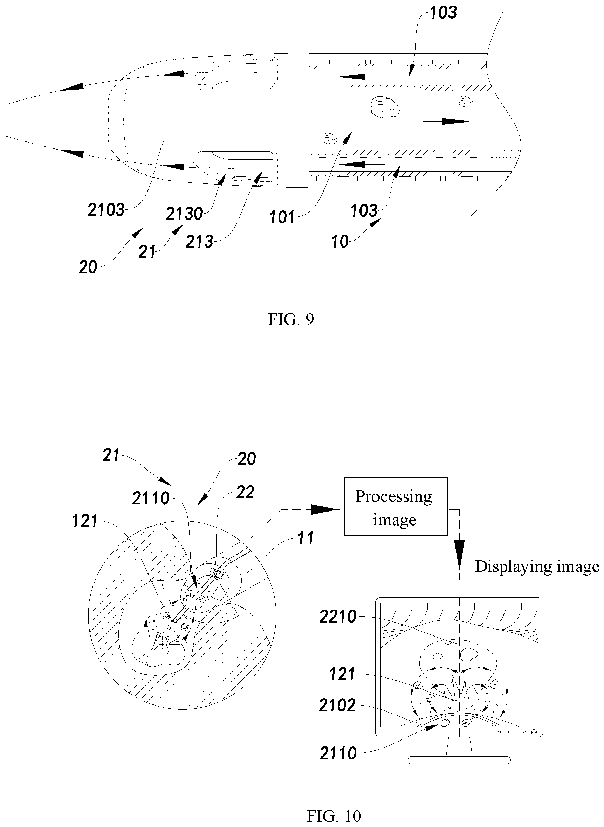

According to the above embodiment of the present application, as shown in FIG. 5 and FIG. 9, the ureteroscope body 10 of the visual ureteroscope 1 may further include a perfusion channel 103 configured to deliver a perfusion liquid (such as water, etc.), and the tip portion 21 of the ureteroscope tip 20 further is provided with a perfusion hole 213 in communication with the perfusion channel 103 to discharge the perfusion liquid delivered through the perfusion channel 103 from the tip portion 21 to be perfused into a human organ. In this way, when the visual ureteroscope 1 is operated, after the visual ureteroscope 1 is inserted into the kidney, the perfusion liquid such as water can flow to the perfusion hole 213 of the tip portion 21 through the perfusion channel 103, and then enter the kidney through the perfusion hole 213 to achieve the perfusion operation. The operating component 12, such as the optical fiber 121, extends from the operating channel 102 to the operating hole 212 to extend out of the suction opening 2110 of the suction hole 211 to perform the calculus crushing operation, and at the same time, excess perfusion liquid and crushed calculus can flow from the suction hole 211 to the suction channel 101 to be discharged from the body.

Preferably, as shown in FIG. 9 and FIG. 10, the perfusion hole 213 of the tip portion 21 extends from the rear end surface of the tip portion 21 to an outer peripheral side surface 2103 of the tip portion 21, so as to form one or more perfusion openings 2130 at the outer peripheral side surface 2103 of the tip portion 21, so that the perfusion liquid can flow outward from the outer peripheral side surface 2103 of the tip portion 21 through the perfusion opening 2130 of the perfusion hole 213, so as to form a controllable and orderly fluid circulation in front of the tip portion 21, which helps to drive the crushed calculus to the suction opening 2110 in all directions for efficient suction.

It should be understood that, according to the law of conservation of momentum and the principle of negative pressure suction in fluid mechanics, the kinetic energy of the perfusion liquid during high-speed flowing is used to push the heavy fragmented calculus (crushed calculus) deposited at the bottom of the renal pelvis to generate displacement, the calculus change the direction after encountering the obstruction of the inner cavity surface of the renal pelvis, and then move upward along the inner wall of the renal pelvis. When reaching the front of the suction opening 2110, the pressure in the vicinity of the suction opening 2110 is relatively low, and the perfusion liquid is forced to flow to the suction opening 2110, so as to drive the crushed calculus to enter the suction opening 2110 until it is discharged from the body. The continuous liquid perfusion and suction in this process can enable the perfusion liquid to form a continuous circular motion track (vortex) of approximate semicircular circumference between the perfusion opening 2130 and the suction opening 2110. The diameter or motion track of the semicircular circumference can be controlled by adjusting the flow rate and the strength of the suction force, so as to achieve targeted and controllable suction of the crushed calculus, and greatly improve the calculus discharging efficiency.

In addition, since an area of the outer peripheral side surface 2103 of the tip portion 21 is relatively large, a number and size of the perfusion opening 2130 of the perfusion hole 213 need not be limited by the end surface of the tip portion 21 with a small area, so that the effective area of the perfusion opening 2130 of the perfusion hole 213 is greatly increased, which helps to form a large perfusion flow under a relatively low perfusion pressure and a larger suction flow in the suction hole 211 under the same negative pressure, thereby achieving an optimal perfusion suction ratio and enhancing calculus discharging efficiency.

Exemplarily, in the first and second embodiments of the present application, as shown in FIG. 11 and FIG. 12, the perfusion channel 103 of the ureteroscope body 10 has an annular structure to wrap the suction channel 101 and the operating channel 102.

It should be noted that, in the above embodiment of the present application, the perfusion channel 103 in the ureteroscope body 10 is independent from the suction channel 101 and the operating channel 102, respectively. In other embodiments of the present application, the perfusion channel 103 may also be in communication with the operating channel 102.

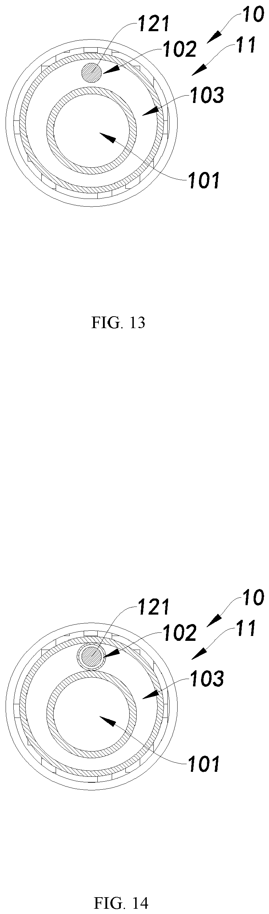

Exemplarily, in a third example of the present application, as shown in FIG. 13, the operating channel 102 and the perfusion channel 103 in the ureteroscope body 10 may also be in communication with each other, that is, the operating channel 102 and the perfusion channel 103 of the ureteroscope body 10 are in communication with each other, so as to form a complete annular channel surrounding the suction channel 101, which helps to simplify the structure of the ureteroscope body 10 and reduce the manufacturing cost of the surgical ureteroscope 1.

Certainly, in a fourth embodiment of the present application, as shown in FIG. 14, the perfusion channel 103 of the ureteroscope body 10 may also have an irregular shaped structure, and the operating channel 102 and the perfusion channel 103 are independent from each other. The perfusion channel 103 and the operating channel 102 are located around the suction channel 101 to cooperatively wrap the suction channel 101, so as to increase an effective diameter of the perfusion channel 103 without increasing an outer diameter of the ureteroscope tube 11 of the ureteroscope body 10, and help to increase the perfusion flow. It should be understood that, in this embodiment of the present application, the suction channel 101 of the ureteroscope body 10 may have a circular cross-section or an elliptical cross-section, and the perfusion channel 103 of the ureteroscope body 10 may have a notched annular cross-section to partially wrap the suction channel 101 and form a notch around a periphery of the suction channel 101 to arrange the operating channel 102, so that the perfusion channel 103 and the operating channel 102 cooperatively surround the suction channel 101.

According to the above embodiment of the present application, as shown in FIG. 4, the ureteroscope body 10 of the visual ureteroscope 1 may further include an operating portion 13 provided at a rear end of the ureteroscope tube 11, and the ureteroscope tube 11 may include an insertion portion 111 extending forward from the operating portion 13 and a bendable portion 112 extending forward from the insertion portion 111. The ureteroscope tip 20 is provided at the bendable portion 112 of the ureteroscope tube 11, and the bendable portion 112 of the ureteroscope tube 11 can be operated by the operating portion 13 to be bent or straighten, so that the ureteroscope tip 20 can approach a target position such as a calculus position in the renal pelvis.

In addition, as shown in FIG. 4, the operating portion 13 of the ureteroscope body 10 may include a suction interface 1301 in communication with the suction channel 101, an operating interface 1302 in communication with the operating channel 102, and a perfusion interface 1303 in communication with the perfusion channel 103. The suction interface 1301 of the operating portion 13 is configured to be connected to a suction device, such that water and crushed calculus can be discharged from the suction channel 101 by the suction device. The operating interface 1302 is configured to be connected to the operating component 12, so that the operating component 12 is inserted into the operating channel 102 through the operating interface 1302. The perfusion interface 1303 is configured to be connected to a perfusion device, so as to inject perfusion fluid into the perfusion channel 103 by the perfusion device.

In particular, as shown in FIG. 4, the operating portion 13 of the ureteroscope body 10 may further include an information interface 1304 communicatively connected to the image capturing device 22. The information interface 1304 is configured to be connected to a terminal device such as a display screen, so as to be communicatively connected to the image capturing device 22 and the terminal device, that is, the information acquired by the image capturing device 22 can be processed or displayed by the terminal device.

It should be understood by those skilled in the art that the embodiments of the present disclosure described in the above description and drawings are only examples and do not limit the present disclosure. The object of the present disclosure has been fully and effectively achieved. The functional and structural principles of the present disclosure have been described in the embodiments, and the embodiments of the present disclosure may have any variations or modifications without departing from the principles.

## Claims

1. A visual ureteroscope, comprising:
a ureteroscope body comprising a ureteroscope tube and an operating component mounted to the ureteroscope tube, and the ureteroscope body being provided with a suction channel extending axially along the ureteroscope tube; and
a ureteroscope tip provided on the ureteroscope body, the ureteroscope tip comprising:
a tip portion provided at a front end of the ureteroscope tube, the tip portion having an imaging end surface and a suction end surface located in front of the imaging end surface, wherein the tip portion is provided with a suction hole in communication with the suction channel and an operating hole through which the operating component extends, the suction hole extends axially forward from the suction channel to the suction end surface to form a suction opening, and the operating hole extends obliquely forward, so that the operating component extending out of the operating hole extends obliquely forward out of the tip portion; and
an image capturing device comprising a camera mounted on the imaging end surface of the tip portion, wherein both the suction opening of the suction hole and a head portion of the operating component extending out of the operating hole are located within a field of view of the camera.

2. The visual ureteroscope according to claim 1, wherein the suction end surface of the tip portion extends obliquely forward from the imaging end surface of the tip portion.

3. The visual ureteroscope according to claim 2, wherein the suction end surface of the tip portion extends obliquely downward and forwardly from the imaging end surface.

4. The visual ureteroscope according to claim 3, wherein the suction end surface of the tip portion comprises a concave end surface extending inwardly and arcuately from the imaging end surface and a convex end surface extending outwardly and arcuately from the concave end surface, and the concave end surface of the suction end surface is tangent to the imaging end surface.

5. The visual ureteroscope according to any one of claims 1 to 4, wherein an operating opening of the operating hole of the tip portion faces the suction opening of the suction hole of the tip portion.

6. The visual ureteroscope according to claim 5, wherein a central axis of the operating hole of the tip portion intersects a central axis of the suction hole.

7. The visual ureteroscope according to claim 6, wherein the operating hole of the tip portion extends obliquely from top to bottom.

8. The visual ureteroscope according to claim 5, wherein the operating hole extends obliquely forward and inward from a rear end surface of the tip portion to an inner wall surface of the suction hole, so as to form the operating opening at the inner wall surface of the suction hole.

9. The visual ureteroscope according to claim 8, wherein a central axis of the operating hole of the tip portion is located in a meridian image plane of the camera.

10. The visual ureteroscope according to claim 9, wherein a central axis of the suction hole of the tip portion is located in the meridian image plane of the camera.

11. The visual ureteroscope according to claim 10, wherein an optical axis of the camera intersects the central axis of the operating hole.

12. The visual ureteroscope according to any one of claims 1 to 4, wherein the ureteroscope body is further provided with an operating channel extending axially within the ureteroscope tube, the operating component extends through the operating channel, and the operating hole of the tip portion is in communication with the operating channel of the ureteroscope body.

13. The visual ureteroscope according to claim 12, wherein the suction channel and the operating channel of the ureteroscope body are in communication with each other.

14. The visual ureteroscope according to claim 12, wherein the ureteroscope body further comprises a perfusion channel extending axially in the ureteroscope tube, the tip portion further is provided with a perfusion hole in communication with the perfusion channel, and the perfusion hole extends from a rear end surface of the tip portion to an outer peripheral side surface of the tip portion, so as to form one or more perfusion openings at the outer peripheral side surface of the tip portion.

15. The visual ureteroscope according to claim 14, wherein the perfusion channel of the ureteroscope body has an annular structure to wrap the suction channel and the operating channel.

16. The visual ureteroscope according to claim 14, wherein the perfusion channel of the ureteroscope body has an irregular shaped structure, and the perfusion channel wraps the suction channel.

17. The visual ureteroscope according to claim 16, wherein the perfusion channel and the operating channel of the ureteroscope body are in communication with each other, so as to form a complete annular channel surrounding the suction channel.

18. The visual ureteroscope according to any one of claims 1 to 4, wherein the image capturing device further comprises at least one light source, and the light source and the camera are adjacently mounted to the tip portion.

19. The visual ureteroscope according to any one of claims 1 to 4, wherein the operating component is an optical fiber configured to emit a laser to perform a calculus crushing operation.
